# EUROPÄISCHE PATENTSCHRIFT

(11) **EP 1 068 323 B1**
(45) Veröffentlichungstag und Bekanntmachung des Hinweises auf die Patenterteilung: **23.06.2004**
(21) Anmeldenummer: 99911505.8
(22) Anmeldetag: 25.03.1999
(51) Int. Cl.: C12N 15/29, A01H 5/00, C07H 3/06, C07K 14/415, A61K 39/36

(54) **REKOMBINANTES HAUPTALLERGEN DES POLLENS VON ARTEMISIA VULGARIS (BEIFUSS)**
RECOMBINANT MAJOR ALLERGEN OF THE POLLEN OF ARTEMISIA VULGARIS (MUGWORT)
ALLERGENE PRINCIPAL RECOMBINE DU POLLEN D'$i(ARTEMISIA VULGARIS) (ARMOISE)

(30) Priorität: 26.03.1998 AT 53998
(43) Veröffentlichungstag der Anmeldung: 17.01.2001
(73) Patentinhaber: "BIOMAY" Produktions- und Handels-Aktiengesellschaft, 1030 Wien (AT)
(72) Erfinder: FERREIRA, Fatima, A-5102 Anthering (AT); RICHTER, Klaus, A-5081 Anif (AT); ENGEL, Edwin, A-8773 Kammern (AT); EBNER, Christof, A-2345 Brunn am Gebirge (AT); KRAFT, Dietrich, A-1170 Wien (AT); BREITENBACH, Michael, A-5020 Salzburg (AT); HIMLY, Martin, A-9500 Villach (AT)
(74) Vertreter: Itze, Peter, Dipl.-Ing.
(86) Internationale Anmeldenummer: PCT/AT1999/000081
(87) Internationale Veröffentlichungsnummer: WO 1999/049045

(56) Entgegenhaltungen:
- WO-A-97/05258
- HIRSCHWEHR R ET AL.: "Identification of common allergenic structures in mugwort and ragweed pollen" THE JOURNAL OF ALLERGY AND CLINICAL IMMUNOLOGY, Bd. 101, Nr. 2 Pt1, Februar 1998 (1998-02), Seiten 196-206, XP000857295

## Beschreibung

Die Erfindung bezieht sich auf rekombinante DNA-Moleküle, die für das Allergen Art v 1a codieren, auf ein Verfahren zur Herstellung eines Art v 1 Moleküles, sowie auf einen Vektor und eine transfomierten Wirtszelle.

Beifußpollen ist einer der wichtigsten Verursacher von Allergien in Europa im Spätsommer (1,2). Unter allen jenen Patienten, die an Pollenallergie leiden, ist die Häufigkeit der durch Beifußpollen ausgelösten allergischen Erkrankungen etwa 10-14% (2,3). Immunblots des Gesamtproteins aus dem Extrakt von Beifußpollen zeigen, daß die IgEs der Patienten ein Hauptallergen vom Molekulargewicht 27-29 kDa erkennen, welches daher Art v 1 genannt wurde. Über 95% aller Patienten, die gegen Beifußpollen allergisch sind, erkennen Art v 1 im IgE-Immunblot. Solche Immunblots werden nachfolgend "Patientenblots" genannt. Einige andere Proteine des Beifußpollens wandern in der SDS-Polyacrylamid-Gelelektrophorese ebenfalls mit einem scheinbaren Molgewicht von 27-29 kDa. Es war daher schwierig einen cDNA-Klon zu isolieren und zu beweisen, daß er für Art v 1 codiert.

Der Erfindung liegt die Aufgabe zugrunde, ein rekombinantes DNA-Molekül zu schaffen, das für das Allergen des Pollens von Artemisia vulgaris codiert.

Erfindungsgemäß wird dies dadurch erreicht, daß ein DNA-Molekül rekombinant geschaffen ist, welches für das Allergen Art v 1a codiert, welches die in SEQ ID Nr. 2 gezeigte Sequenz besitzt. Damit ist das Hauptallergen von Artemisia vulgaris aufgefunden und einer Diagnose bzw. Therapie zugänglich gemacht. Diese DNA-Moleküle sind durch die Nukleotidsequenz gemäß SEQ ID Nr.1 gekennzeichnet. Diese Moleküle können aber auch eine durch Degeneration des genetischen Codes aus der Aminosäuresequenz gemäß SEQ ID NR. 2 abgeleitet sein. Bevorzugt können die erfindungsgemäßen Moleküle mehr als 60 % Sequenzidentität mit SEQ ID Nr. 1 aufweisen. Weiters kann das erfindungsgemäße rekombinante DNA-Molekül für die in SEQ ID Nr. 4 und 6 gezeigten Aminosäuresequenzen der Isoformen Art v 1b und Art v 1c codieren. Dabei können diese rekombinanten DNA-Moleküle die in SEQ ID Nr. 3 und 5 gezeigten Nukleotidsequenzen aufweisen. Die erfindungsgemäßen rekombinanten DNA-Moleküle können mit der in SEQ ID Nr. 1 gezeigten Sequenz hybridisieren und unter stringenten Waschbedingungen durch Hybridisierung gebunden bleiben.

Gleiches gilt auch hinsichtlich der in SEQ ID Nr. 3 und 5 gezeigten Sequenzen. Als stringente Hybridisierungsbedingungen sind z. B. 1 M NaCl in H₂O bei 60°C und als stringente Waschbedingungen sind z. B. 2 Mal für 30 min. waschen bei 50°C in 5x SSPE und 0,1% SDS anzusehen (1x SSPE ist 0,18 M NaCl 0,01 M Natriumphosphat pH 7,4, 1m M EDTA).

Ein erfindungsgemäßes Verfahren zur Herstellung eines Art v1 Allergens ist durch die folgenden Schritte gekennzeichnet:
a) Züchten prokaryotischer oder eukaryotischer Wirtszellen, die eine für Art v 1 codierende DNA (SEQ ID Nr. 1) bzw. eine mit dieser Sequenz 60 %ige Sequenzidentität aufweisende DNA enthalten, so daß das Art v 1 Allergen durch die Wirtszellen exprimiert wird;
b) Isolierung des Allergens Art v 1.

Dieses rekombinante Allergen kann glycosyliert sein. Für das Verfahren kann ein replikationsfähiger prokaryolischer oder eukaryotischer Expressionssektor vorgesehen sein, der die unter Schritt a) des Verfahrens angeführten DNA-Moleküle enthält. Ein solcher Expressionssektor ist in den genannten Wirtszellen enthalten, welche bevorzugt Escherichia coli oder Pichia pastoris sein können.

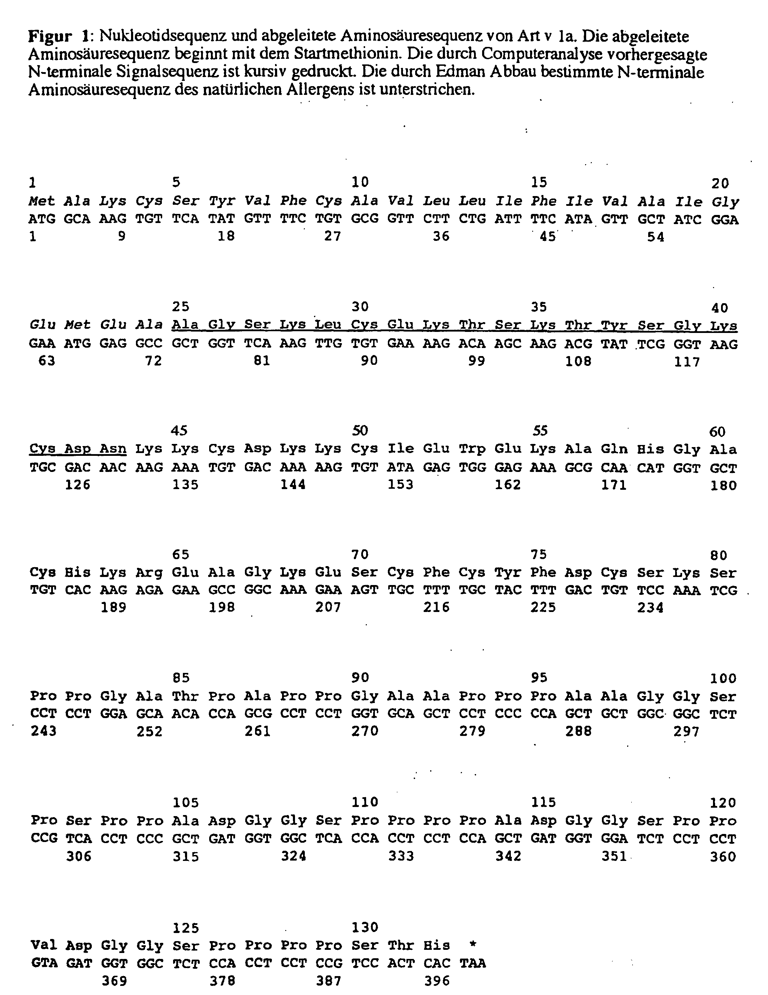

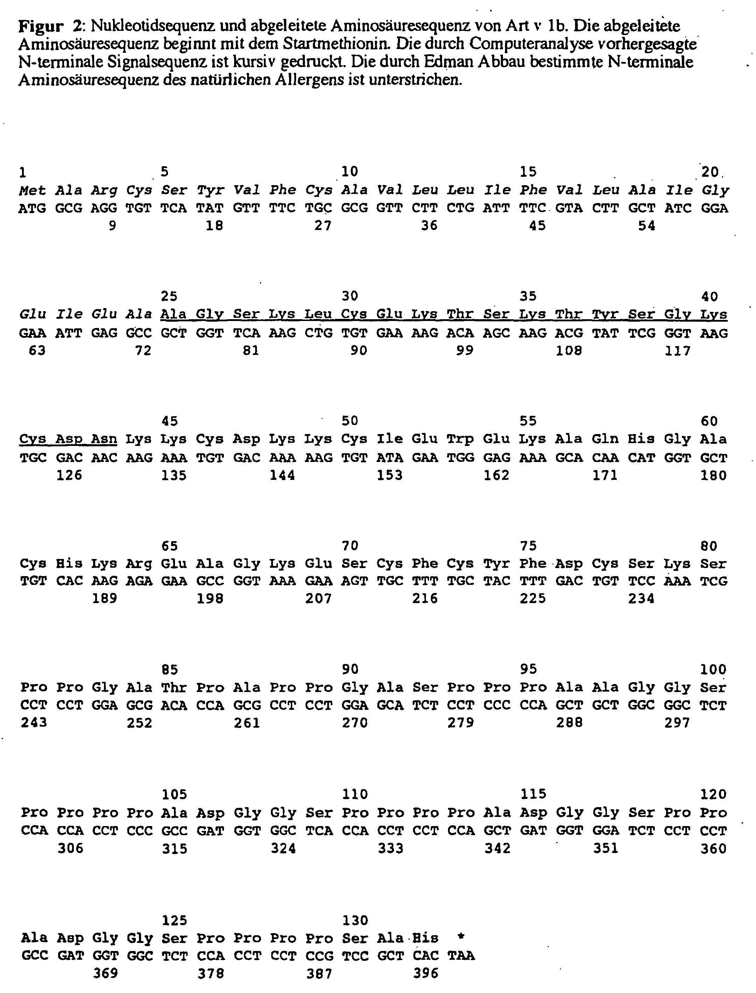

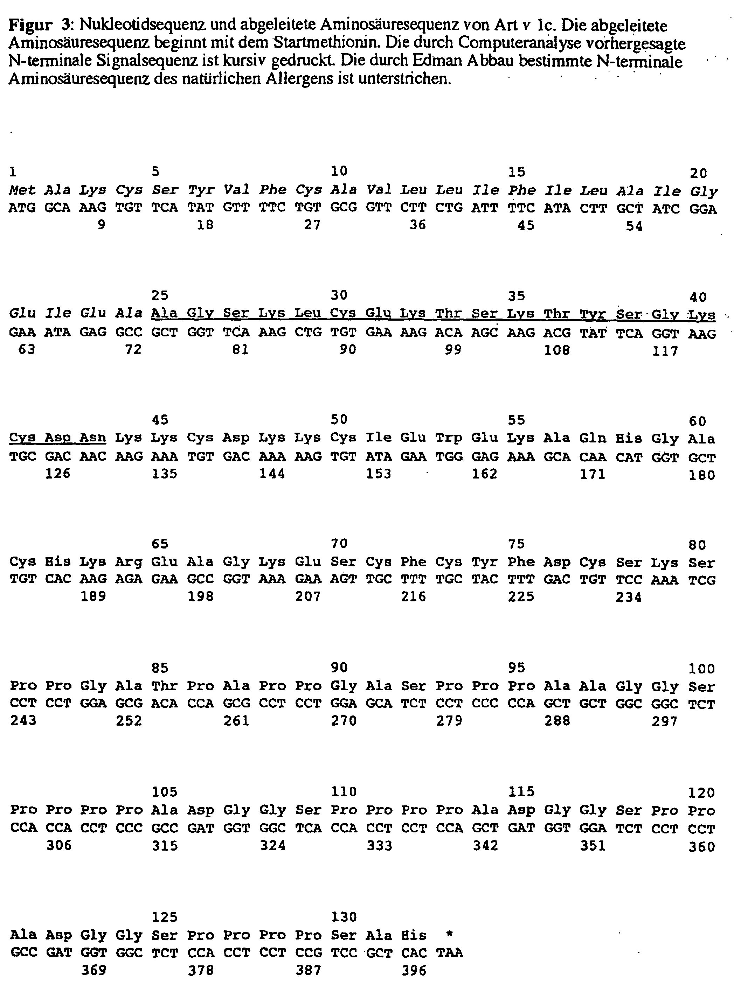

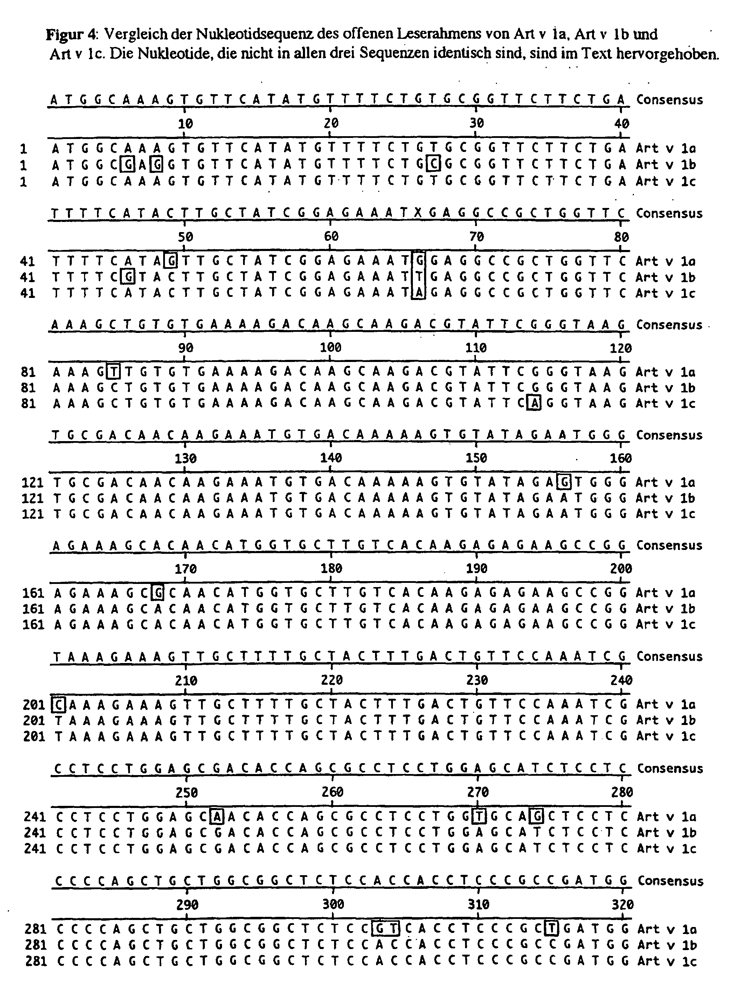

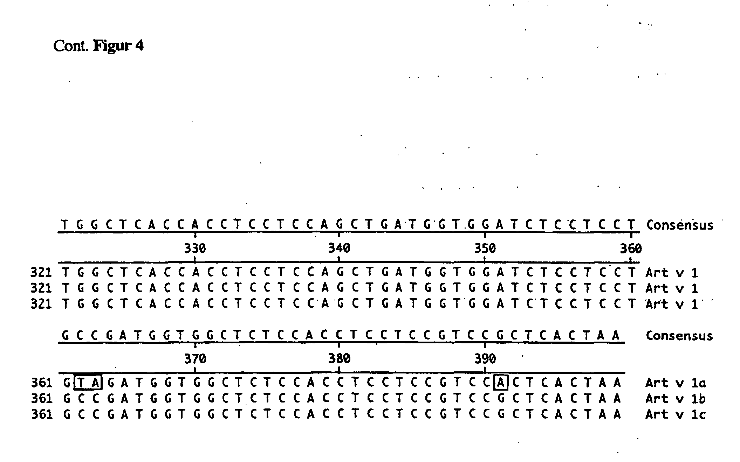

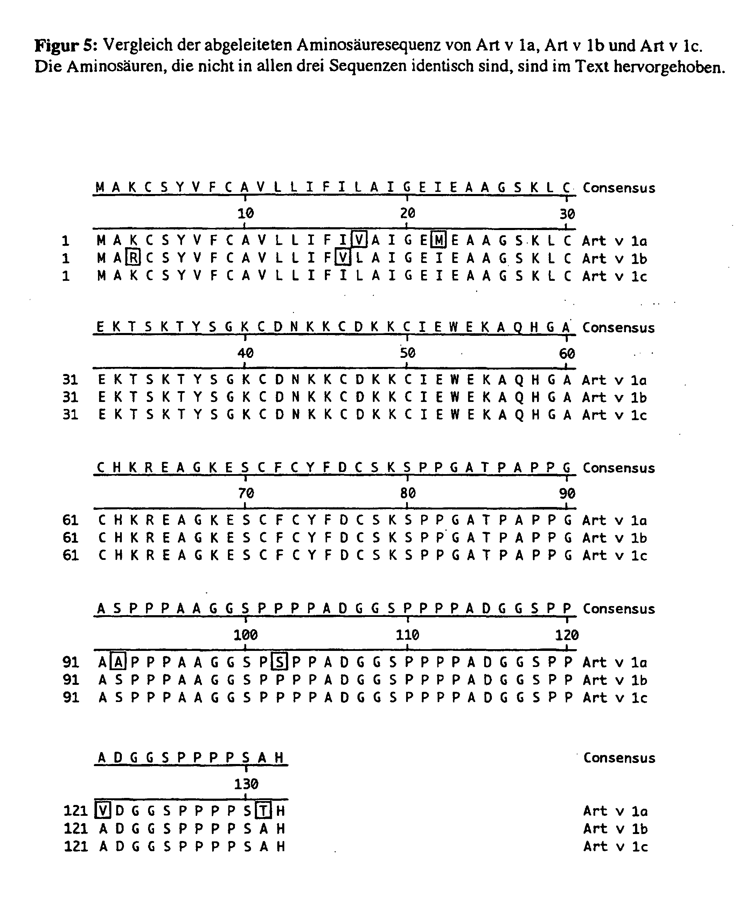

Es wird also die Isolierung eines authentischen und vollständigen cDNA-Klons gezeigt, der für Art v 1a (Fig. 1), das Hauptallergen des Beifußpollens codiert. Der Buchstabe a in Art v 1a bezeichnet die Isoform a. Dazu wurden zwei weitere Klone isoliert, die ebenfalls für authentische und vollständige Isoformen von Art v 1 codieren, die Art v 1b und Art v 1c genannt wurden. Die Sequenzen der beiden letztgenannten Isoformen werden in Fig. 2 und Fig. 3 gezeigt. Die Klone sind am 5'-Ende vollständig, was daraus ersichtlich ist, daß sie ein AUG Startcodon in einem typisch eukaryotischen Kontext beinhalten. Die Klone sind aber auch am 3'-Ende vollständig, da sie nach dem Stopcodon 177-200 Nukleotide gefolgt von einer polyA-Sequenz enthalten. Die außerhalb der offenen Leserahmen liegenden Sequenzen der Klone werden in den Abbildungen nicht gezeigt. Die beiden Fig. 4 und 5 zeigen "alignments" der Nukleotidsequenzen und der abgeleiteten Aminosäuresequenzen von Art v 1a, b und c. Diese Vergleiche zeigen, daß Art v 1 eine Mischung verschiedener Isoformen ist, die untereinander verhältnismäßig große Abweichungen sowohl der Nukleotid- als auch der Aminosäuresequenz zeigen. Diese Sequenzabweichungen sind in den nichttranslatierten Bereichen der cDNA-Sequenzen größer als innerhalb der offenen Leserahmen (Daten nicht gezeigt). Sehr wahrscheinlich werden die Isoformen von Art v 1 von einer Genfamilie codiert. Diese Genfamilie ist allerdings nicht spezifisch für die Art oder Gattung von Artemis vulgaris, da eine homologe cDNA-Sequenz auch in der Sonnenblume gefunden wurde (4). Dieses Protein der Sonnenblume (SF18) wird in den Epidermiszellen der Antheren synthetisiert und könnte eine Pollen-spezifische Funktion haben. Es zeigt Ähnlichkeit zur Familie der Gamma-Purothionine.

### BEISPIEL:

Die Isolierung des für Art v 1a codierenden Klons wurde in folgender Weise durchgeführt:

Es wurde eine cDNA Expressionsbank in dem Phagen Lambda-ZAP II (Stratagene, La Jolla, California; Katalog-Nr.237612) hergestellt. Das Ausgangsmaterial war die aus Beifußpollen isolierte mRNA. Diese cDNA-Bank wurde mit einem Serumgemisch gescreent, das aus 20 Seren von Patienten bestand, welche im Patientenblot Art v 1 erkennen. Ein Klon, welcher positiv mit dem Serumgemisch reagierte, wurde weiter untersucht und ergab schließlich 100% immunopositive Plaques. Die Immunreaktion dieses Klons war relativ schwach, aber eindeutig positiv. Der Beweis, daß dieser Klon für Art v 1 codiert, wurde wie folgt geführt:

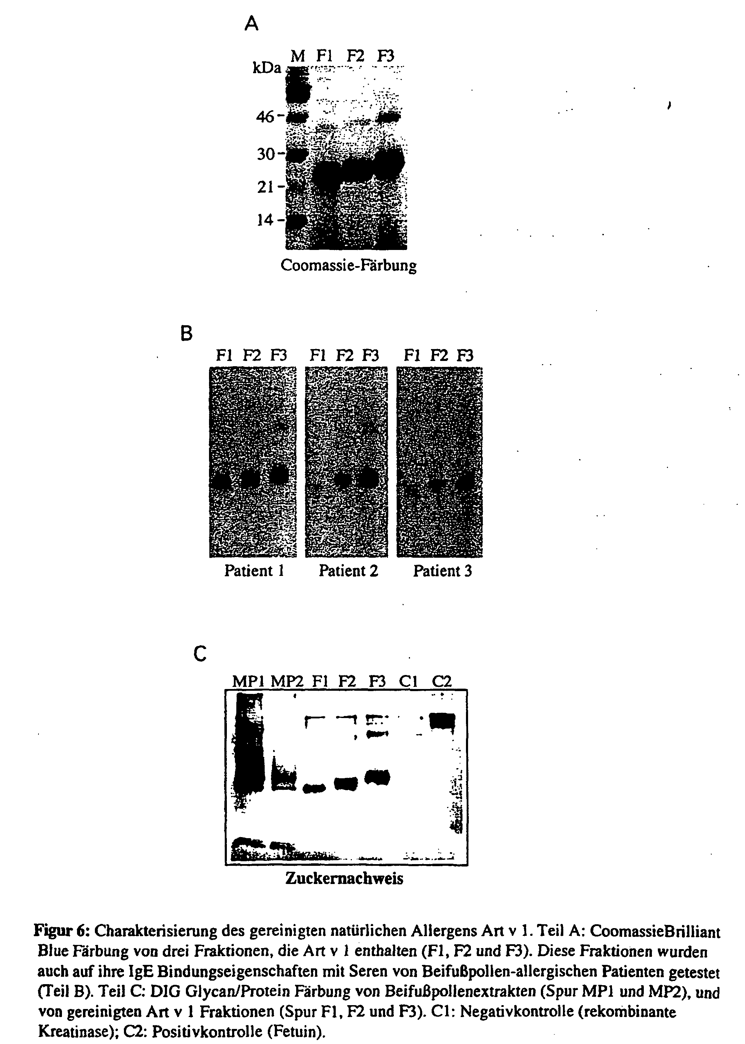

Das Hauptallergen Art v 1 wurde unter sehr milden Bedingungen, nämlich Raumtemperatur, Extraktion mit Wasser für 15-30 min. unter leichtem Schütteln, aus Beifußpollen extrahiert. Dieser Extrakt wurde durch präparative Gelelektrophorese weiter aufgetrennt und die Fraktionen wurden im Immunblot getestet. Fig. 6a zeigt drei Fraktionen (F1, F2 und F3), die nach der Färbung mit Coomassie Brilliant Blue frei von Proteinverunreinigungen waren und Proteinbanden mit einem scheinbaren Molekulargewicht zwischen 22 und 29 kDa zeigten. Gelegentlich war auch eine Dimerenbande bei 50 kDa erkennbar. Fig. 6b zeigt die Patientenblots dieser Fraktionen. Es ist klar, daß die Fraktion mit dem höchsten Molekulargewicht Seren von allen 3 hier getesteten Patienten bindet, während die Fraktion mit dem geringsten Molekulargewicht von den Patientenseren nur schwach erkannt wird. Fig. 6c zeigt die selben Proteinblots nach Färbung mit dem "glycoprotein detection kit" (DIG glycan/protein double labeling kit, Katalog Nr. 1500783) von Boehringer Mannheim. Es zeigt sich klar, daß alle drei Fraktionen aus Glycoprotein bestehen. Alle drei Fraktionen wurden durch N-terminalen Edman Abbau charakterisiert und ergaben identische N-terminale Sequenzen, welche in den Fig. 1- 3 unterstrichen sind. Die Computeranalyse der abgeleiteten Proteinsequenz in Fig. 1 nach Nielsen et al. (5) sagt vorher, daß Art v 1 eine typische N-terminale hydrophobe Signalsequenz enthält, die zum "targeting" ins endoplasmatische Retikulum und in den Golgiapparat führt. Die Sequenz nach dem Beginn der durch die Computeranalyse vorhergesagten reifen Proteinsequenz ist identisch mit der im natürlichen Protein gefunden N-terminalen Sequenz (unterstrichen in den Fig. 1-3). Eine ganz ähnliche N-terminale partielle Sequenz wurde von Matthiesen et al. (6) gefunden. Es kann daher daraus geschlossen werden, daß Art v 1 ein sezerniertes Glycoprotein ist, dessen N-terminus durch die Abspaltung einer typischen ER Signalsequenz entsteht. Das natürliche Protein ist durch Unterschiede im Glycosilierungsgrad heterogen, und die voll glycosilierte Form (F3 in Fig. 6) bindet IgE am besten.

Der erste auf diese Weise isolierte cDNA-Klon von Art v 1 wurde nun mit Phosphor 32 markiert und als Hybridisierungsprobe benützt. Die oben erwähnte cDNA-Klonbank wurde mit dieser Hybridisierungsprobe gescreent und zwei weitere Klone, Art v 1b und Art v 1c, wurden gefunden. Diese Klone wurden durch sequenzieren der DNA weiter analysiert. Die Sequenzen werden in den Figs. 2 und 3 gezeigt.

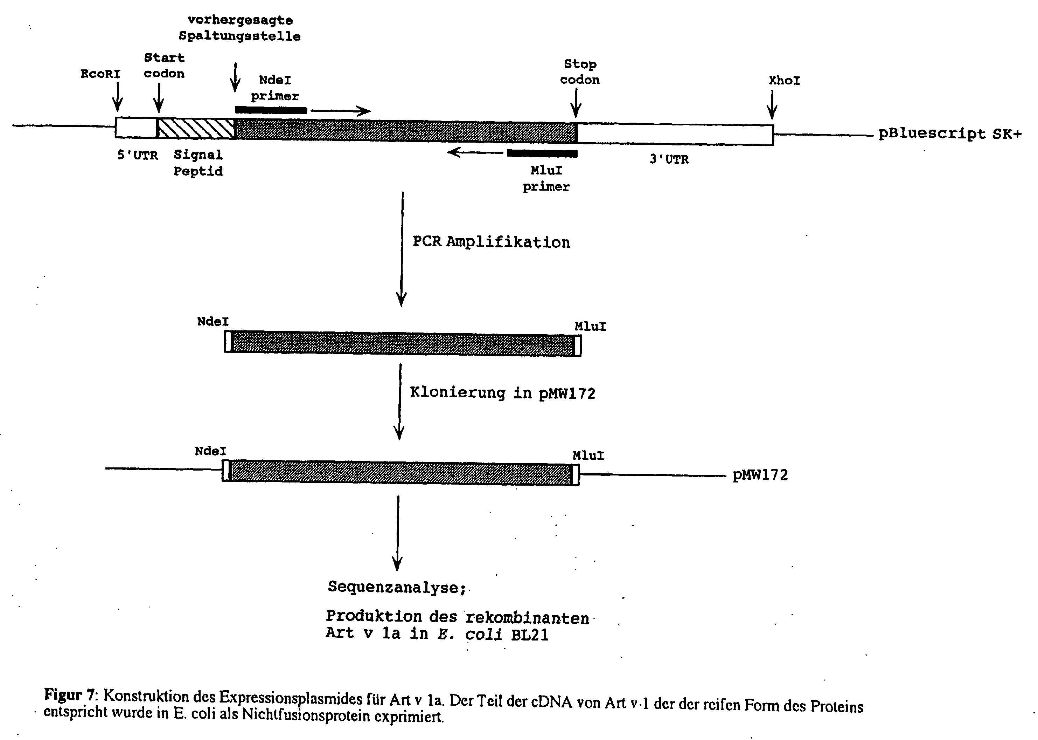

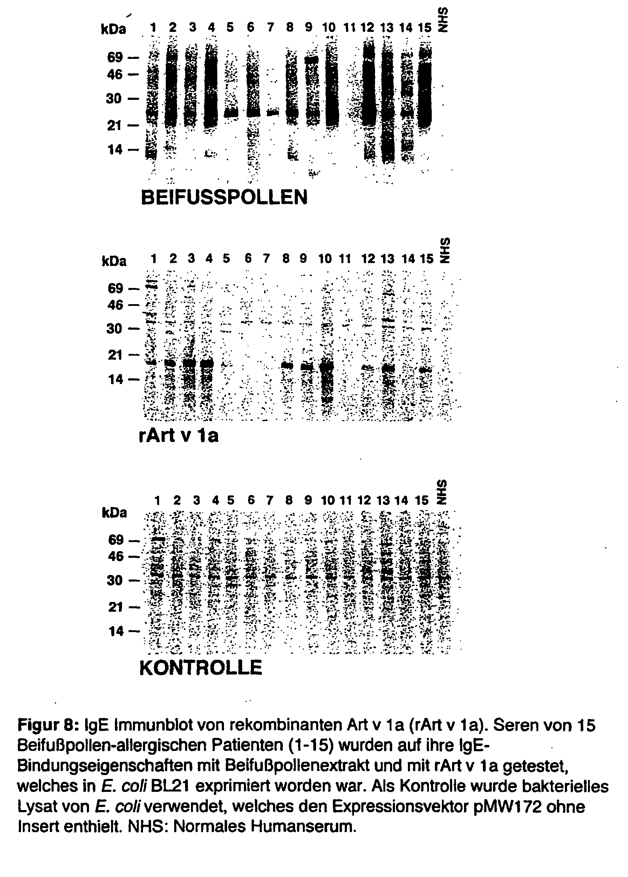

Es wurde E. coli benutzt, um die reife (kurze) Form von Art v 1a als rekombinantes Nichtfusionsprotein zu exprimieren. Dazu wurde der entsprechende Teil der cDNA in das Expressionssystem pMW172 cloniert. Es ist wohl bekannt, daß in E. coli exprimierte rekombinante Proteine keine postsynthetischen Modifikationen aufweisen, mit der möglichen Ausnahme der Abspaltung des N-terminalen Methionins. Solche rekombinanten Proteine enthalten keine Zuckermoleküle. Die Konstruktion des Expressionsplasmids wird in Fig. 7 gezeigt. Das rekombinante Protein Art v 1 reicherte sich in der löslichen Fraktion der E. coli Proteine an. Fig. 8 zeigt Patientenblots der löslichen Fraktion mit 15 Patienten. Das scheinbare Molekulargewicht des natürlichen Proteins beträgt etwa 27 kDa (Fig. 8 Beifußpollen), während das scheinbare Molekulargewicht des rekombinanten Proteins wegen der Abwesenheit der Zucker nur 18 kDa beträgt. Das theoretische Molekulargewicht wäre 10,8 kDa, was darauf hinweist, daß das rekombinante Protein eine sehr ungewöhnliche elektrophoretische Mobilität in der SDS-Polyacrylamid-Gelelektrophorese zeigt. Fig. 8 (rArt v 1a) zeigt sehr deutlich, daß 10 der 15 Patienten im Patientblot das unglycosilierte Protein erkennen. Es existieren aber auch Patienten, die diese Form des Proteins nicht erkennen. Überraschenderweise erkennen die Patienten 3, 4 und 10 die unglycosilierte Form besser als die natürliche glycosilierte Form. Die Kontrollen in Fig. 8 zeigen das E. coli Proteine von den Patienten und von den sekundären Antikörpern nicht oder nur ganz schwach erkannt werden. Diese sehr schwachen Banden zeigen sich gleichmäßig in allen drei in Fig. 8 gezeigten Patientenblots.

Um die im letzten Absatz vorgestellten experimentellen Ergebnisse zu erklären, wird folgende Hypothese aufgestellt: Es könnte sein, daß die Zuckeranteile von Art v 1 notwendig sind, um das Proteinrückgrat in seine natürliche Konformation zu bringen, so daß die IgE einiger Patienten an diese so entstandenen Epitope binden können. Einige andere Epitope sind hingegen in Abwesenheit der Zucker gut für die Patientenseren erkennbar. Eine andere aber weniger wahrscheinliche Erklärung für diese Beobachtungen besteht in der Möglichkeit, daß die Seren jener Patienten, die die unglycosilierte Form nicht erkennen, tatsächlich direkt gegen Epitope gerichtet sind, die aus den Zuckermolekülen bestehen.

### Vorläufige Charakterisierung des Zuckeranteils des natürlichen Hauptallergens von Artemisia vulgaris, Art v 1.

Natürliches Hauptallergen von Artemisia vulgaris wurde zur Homogenität gereinigt durch:
1. Anionenaustauschchromatographie an Sepharose Q (Pharmacia, Uppsala, Schweden) bei pH=5.2 und
2. HPLC-Gelfiltrationschromatographie an TSK-Gel G2000SW (Tosohaas, Stuttgart, BRD).

Das Material war homogen hinsichtlich seiner N-terminalen Proteinsequenz und Aminosäurezusammensetzung, jedoch heterogen hinsichtlich des Molekulargewichtes durch unterschiedliche Glykosylierung. Die Bestimmung des Molekulargewichtes erfolgte durch MALDI-TOF Massenspektrometrie und ergab zwei breite Peaks mit den mittleren Molekülmassen 13.5 kD und 15.5 kD. Das durch SDS-PAGE ermittelte scheinbare Molekulargewicht war jedoch 24 - 28 kD, was auf eine sehr ungewöhnliche Proteinstruktur oder ungewöhnliche Struktur des Glykoanteils hinweist. Die vorläufige Analyse der kovalent am Protein gebundenen Zucker durch Hydrolyse und HPLC ergab, daß keine N-Glykosylierung und sehr wahrscheinlich auch keine typische O-Glykosylierung vorliegt, sondern wahrscheinlich eine bei pflanzlichen Glykoproteinen schon früher beschriebene Glykosylierung an Hydroxyprolin. Das sehr prolinreiche Protein, Art v 1 (20% Prolin) ist postsynthetisch modifiziert, so daß im reifen Protein Prolin und Hydroxyprolin im Verhätlnis 4:6 vorliegen. Ausgedehnte Studien mit fünf verschiedenen Lektinen (Galanthus nivalis Agglutinin, Sambucus nigra Agglutinin, Maackia amurensis Agglutinin, Peanut Agglutinin und Datura stramonium Agglutinin), die für bekannte Zuckerstrukturen der N-Glykosylierung und O-Glykosylierung spezifisch sind, zeigten, daß diese Strukturen in Art v 1 nicht vorhanden sind. Die Rolle dieser ungewöhnlichen Proteinstruktur und der Struktur des Glykoanteils für die Ausbidung der B-Zell-Epitope dieses Allergens wird zur Zeit intensiv untersucht. Fig. 8 gibt einen ersten Eindruck davon, daß bei diesem Allergen die Glykoanteile eine wichtige Rolle für die IgE-Bindung spielen könnten.

### SEQUENZPROTOKOLLE:

(iii) ZAHL DER SEQUENZEN: 6

(2) ANGABEN ZU SEQ ID NO:1:
   (i) SEQUENZKENNZEICHEN:
      (A) LÄNGE: 399 Basenpaare
      (B) ART: Nukleotidsequenz
      (C) STRANGFORM: Doppelstrang
      (D) TOPOLOGIE: linear
   (ii) ART DES MOLEKÜLS: c-DNA zu m-RNA
   (iii) HYPOTHETISCH: Nein
   (iv) ANTISENSE: Nein
   (vi) URSPRÜNGLICHE HERKUNFT:
      (A) ORGANISMUS: Artemisia vulgaris
      (D) ENTWICKLUNGSSTADIUM:Pollen
   (xi) SEQUENZBESCHREIBUNG: SEQ ID NO:1:
   ATGGCAAAGT GTTCATATGT TTTCTGTGCG GTTCTTCTGA TTTTCATAGT TGCTATCGGA    60
   GAAATGGAGG CCGCTGGTTC AAAGTTGTGT GAAAAGACAA GCAAGACGTA TTCGGGTAAG   120
   TGCGACAACA AGAAATGTGA CAAAAAGTGT ATAGAGTGGG AGAAAGCGCA ACATGGTGCT   180
   TGTCACAAGA GAGAAGCCGG CAAAGAAAGT TGCTTTTGCT ACTTTGACTG TTCCAAATCG   240
   CCTCCTGGAG CAACACCAGC GCCTCCTGGT GCAGCTCCTC CCCCAGCTGC TGGCGGCTCT   300
   CCGTCACCTC CCGCTGATGG TGGCTCACCA CCTCCTCCAG CTGATGGTGG ATCTCCTCCT   360
   GTAGATGGTG GCTCTCCACC TCCTCCGTCC ACTCACTAA                          399
(2) ANGABEN ZU SEQ ID NO:2:
   (i) SEQUENZKENNZEICHEN:
      (A) LÄNGE: 132 Aminosäuren
      (B) ART: Aminosäure
      (C) STRANGFORM: Einzelstrang
      (D) TOPOLOGIE: unbekannt
   (ii) ART DES MOLEKÜLS: Protein
   (iii) HYPOTHETISCH: Nein
   (iv) ANTISENSE: Nein
   (vi) URSPRÜNGLICHE HERKUNFT:
      (A) ORGANISMUS: Artemisia vulgaris
      (D) ENTWICKLUNGSSTADIUM: Pollen
   (xi) SEQUENZBESCHREIBUNG: SEQ ID NO:2:
   Met Ala Lys Cys Ser Tyr Val Phe Cys Ala Val Leu Leu Ile Phe
   1               5                   10                  15
   Ile Val Ala Ile Gly Glu Met Glu Ala Ala Gly Ser Lys Leu Cys
                   20                  25                  30
   Glu Lys Thr Ser Lys Thr Tyr Ser Gly Lys Cys Asp Asn Lys Lys
                   35                  40                  45
   Cys Asp Lys Lys Cys Ile Glu Trp Glu Lys Ala Gln His Gly Ala
                   50                  55                  60
   Cys His Lys Arg Glu Ala Gly Lys Glu Ser Cys Phe Cys Tyr Phe
                   65                  70                  75
   Asp Cys Ser Lys Ser Pro Pro Gly Ala Thr Pro Ala Pro Pro Gly
                   80                  85                  90
   Ala Ala Pro Pro Pro Ala Ala Gly Gly Ser Pro Ser Pro Pro Ala
                   95                  100                 105
   Asp Gly Gly Ser Pro Pro Pro Pro Ala Asp Gly Gly Ser Pro Pro
                   110                 115                 120
   Val Asp Gly Gly Ser Pro Pro Pro Pro Ser Thr His
                   125                 130
(2) ANGABEN ZU SEQ ID NO:3:
   (i) SEQUENZKENNZEICHEN:
      (A) LÄNGE: 399 Basenpaare
      (B) ART: Nukleotidsequenz
      (C) STRANGFORM: Doppelstrang
      (D) TOPOLOGIE: linear
   (ii) ART DES MOLEKÜLS: c-DNA zu m-RNA
   (iii) HYPOTHETISCH: Nein
   (iv) ANTISENSE: Nein
   (vi) URSPRÜNGLICHE HERKUNFT::
      (A) ORGANISMUS: Artemisia vulgaris
      (D) ENTWICKLUNGSSTADIUM: Pollen
   (xi) SEQUENZBESCHREIBUNG: SEQ ID NO:3:
   ATGGCGAGGT GTTCATATGT TTTCTGCGCG GTTCTTCTGA TTTTCGTACT TGCTATCGGA    60
   GAAATTGAGG CCGCTGGTTC AAAGCTGTGT GAAAAGACAA GCAAGACGTA TTCGGGTAAG   120
   TGCGACAACA AGAAATGTGA CAAAAAGTGT ATAGAATGGG AGAAAGCACA ACATGGTGCT   180
   TGTCACAAGA GAGAAGCCGG TAAAGAAAGT TGCTTTTGCT ACTTTGACTG TTCCAAATCG   240
   CCTCCTGGAG CGACACCAGC GCCTCCTGGA GCATCTCCTC CCCCAGCTGC TGGCGGCTCT   300
   CCACCACCTC CCGCCGATGG TGGCTCACCA CCTCCTCCAG CTGATGGTGG ATCTCCTCCT   360
   GCCGATGGTG GCTCTCCACC TCCTCCGTCC GCTCACTAA                          399
(2) ANGABEN ZU SEQ ID NO:4:
   (i) SEQUENZKENNZEICHEN:
      (A) LÄNGE: 132 Aminosäuren
      (B) ART: Aminosäure
      (C) STRANGFROM: Einzelstrang
      (D) TOPOLOGIE: unbekannt
   (ii) ART DES MOLEKÜLS: Protein
   (iii) HYPOTHETISCH: Nein
   (iv) ANTISENSE: Nein
   (vi) URSPRÜNGLICHE HERKUNFT:
      (A) ORGANISMUS: Artemisia vulgaris
      (D) ENTWICKLUNGSSTADIUM: Pollen
   (xi) SEQUENZBESCHREIBUNG: SEQ ID NO:4:
   Met Ala Arg Cys Ser Tyr Val Phe Cys Ala Val Leu Leu Ile Phe
   1               5                   10                  15
   Val Leu Ala Ile Gly Glu Ile Glu Ala Ala Gly Ser Lys Leu Cys
                   20                  25                  30
   Glu Lys Thr Ser Lys Thr Tyr Ser Gly Lys Cys Asp Asn Lys Lys
                   35                  40                  45
   Cys Asp Lys Lys Cys Ile Glu Trp Glu Lys Ala Gln His Gly Ala
                   50                  55                  60
   Cys His Lys Arg Glu Ala Gly Lys Glu Ser Cys Phe Cys Tyr Phe
                   65                  70                  75
   Asp Cys Ser Lys Ser Pro Pro Gly Ala Thr Pro Ala Pro Pro Gly
                   80                  85                  90
   Ala Ser Pro Pro Pro Ala Ala Gly Gly Ser Pro Pro Pro Pro Ala
                   95                  100                 105
   Asp Gly Gly Ser Pro Pro Pro Pro Ala Asp Gly Gly Ser Pro Pro
                   110                 115                 120
   Ala Asp Gly Gly Ser Pro Pro Pro Pro Ser Ala His
                   125                 130
(2) ANGABEN ZU SEQ ID NO:5:
   (i) SEQUENZKENNZEICHEN:
      (A) LÄNGE: 399 Basenpaare
      (B) ART: Nukleotidsequenz
      (C) STRANGFORM: Doppelstrang
      (D) TOPOLOGIE: linear
   (ii) ART DES MOLEKÜLS: c-DNA zu m-RNA
   (iii) HYPOTHETISCH: Nein
   (iv) ANTISENSE: Nein
   (vi) URSPRÜNGLICHE HERKUNFT:
      (A) ORGANISMUS: Artemisia vulgaris
      (D) ENTWICKLUNGSSTADIUM: Pollen
   (xi) SEQUENZBESCHREIBUNG: SEQ ID NO:5:
   ATGGCAAAGT GTTCATATGT TTTCTGTGCG GTTCTTCTGA TTTTCATACT TGCTATCGGA    60
   GAAATAGAGG CCGCTGGTTC AAAGCTGTGT GAAAAGACAA GCAAGACGTA TTCAGGTAAG   120
   TGCGACAACA AGAAATGTGA CAAAAAGTGT ATAGAATGGG AGAAAGCACA ACATGGTGCT   180
   TGTCACAAGA GAGAAGCCGG TAAAGAAAGT TGCTTTTGCT ACTTTGACTG TTCCAAATCG   240
   CCTCCTGGAG CGACACCAGC GCCTCCTGGA GCATCTCCTC CCCCAGCTGC TGGCGGCTCT   300
   CCACCACCTC CCGCCGATGG TGGCTCACCA CCTCCTCCAG CTGATGGTGG ATCTCCTCCT   360
   GCCGATGGTG GCTCTCCACC TCCTCCGTCC GCTCACTAA                          399
(2) ANGABEN ZU SEQ ID NO:6:
   (i) SEQUENZKKENNZEICHEN:
      (A) LÄNGE: 132 Aminosäuren
      (B) ART: Aminosäure
      (C) STRANGFORM: Einzelstrang
      (D) TOPOLOGIE: unbekannt
   (ii) ART DES MOLEKÜLS: Protein
   (iii) HYPOTHETISCH: Nein
   (iv) ANTISENSE: Nein
   (vi) URSPRÜNGLICHE HERKUNFT:
      (A) ORGANISMUS: Artemisia vulgaris
      (D) ENTWICKLUNGSSTADIUM: Pollen
   (xi) SEQUENZBESCHREIBUNG: SEQ ID NO:6:

### LITERATUR:

1. Charpin, J., Surinyach, R., and Frankland, A.W. (1974) Atlas of European Allergenic pollens. Sandoz, Paris.
2. Spieksma, F.T.M., Charpin, H., Nolard, N., and Stix, E. (1980) Clin. Allergy 10: 319-329.
3. Cornillon, J., Bemard, J-P., Gueho, E., and Touraine, R. (1972) Rev. Franc. Allergol. 12: 131-135.
4. Domon, C., Evrard, J.L., Herdenberger, F., Pillay, D.T.N., and Steinmetz, A. (1990) Plant Mol. Biol. 15: 643-646.
5. Nielsen, H., Engelbrecht, J., Brunak, S., and von Heijne, G. (1997) Prot. Eng. 10: 1-6.
6. Matthiesen, F., Ipsen, H., and Løwenstein, H. (1991) In: Allergenic pollen and pollinosis in Europe. pp 36-44. D'Amato, G., Spieksma, F.T.M., and Bonini, S. eds., Blackwell Scientific Publications, Cambridge.

## Patentansprüche

1. Rekombinante DNA-Moleküle, die für das Allergen Art v 1a codieren, welches die in SEQ ID NO:2 gezeigte Sequenz besitzt.

2. Rekombinante DNA-Moleküle nach Anspruch 1, **dadurch gekennzeichnet, daß** diese DNA-Moleküle die Nukleotidsequenz von SEQ ID NO:1 aufweisen

3. Rekombinante DNA-Moleküle nach Anspruch 1, **dadurch gekennzeichnet, daß** diese Moleküle eine Nukleotidsequenz aufweisen, die aus der Aminosäuresequenz von SEQ ID NO:2 auf Grund der Degeneration des genetischen Codes abgeleitet wurde.

4. Rekombinante DNA-Moleküle nach Anspruch 1, die mehr als 60% Sequenzidentität mit SEQ ID NO: 1 aufweisen.

5. Rekombinante DNA-Moleküle nach Anspruch 4, die für die Isoformen Art v 1b und Art v 1c codieren, **dadurch gekennzeichnet, daß** diese Isoformen die in SEQ ID NO:4 und 6 gezeigten Aminosäuresequenzen aufweisen

6. Rekombinante DNA-Moleküle nach Anspruch 5, **dadurch gekennzeichnet, daß** diese DNA-Moleküle die in SEQ ID NO:3 und 5 gezeigten Nukleotidsequenzen aufweisen.

7. Rekombinante DNA-Moleküle nach Anspruch 1 oder 5, die unter stringenten Bedingungen mit der in SEQ ID NO:1 gezeigten Sequenz hybridisieren und unter stringenten Waschbedingungen durch Hybridisierung gebunden bleiben.

8. Rekombinante DNA-Moleküle nach Anspruch 7, **dadurch gekennzeichnet, daß** die Isoformen Art v 1b und Art v 1c; welche die in SEQ ID NO:3 und 5 gezeigten Nukleotidsequenzen aufweisen, unter stringenten Bedingungen mit der in SEQ ID NO:1 gezeigten Art v 1a Sequenz hybridisieren.

9. Eine Methode für die Produktion des Art v 1 Allergens die aus den folgenden Schritten besteht:
(a) Züchten prokaryotischer oder eukaryotischer Wirtszellen die eine für Art v 1 codierende DNA nach Anspruch 2, 4 oder 7 enthalten, so daß das Art v 1 Allergen durch die Wirtszellen exprimiert wird;
(b) Isolierung des Allergens Art v 1.

10. Eine Methode nach Anspruch 9, **dadurch gekennzeichnet, daß** das rekombinante Allergen glycosiliert ist.

11. Verfahren zur Herstellung einer rekombinanten DNA nach Anspruch 7, **dadurch gekennzeichnet, daß** als stringente Hybridisierungsbedingungen 1 M NaCl in H2O bei 60° C. und als stringente Waschbedingungen 2 Mal für 30 min. waschen bei 50° C in 5x SSPE und 0.1% SDS. 1x SSPE = 0.18M NaCl, 0.01M Natriumphosphat pH = 7.4, 1mM EDTA eingesetzt werden.

12. Ein replikationsfähiger prokaryotischer oder eukaryotischer Expressionsvektor, der DNA-Moleküle entsprechend den Ansprüchen 2, 4 oder 7 als Insert enthält.

13. Eine prokaryotische oder eukaryotische Wirtszelle, die einen Expressionsvektor nach Anspruch 11 enthält.

14. Eine Wirtszelle nach Anspruch 13, **dadurch gekennzeichnet, daß** diese Wirtszelle Escherichia coli ist.

15. Eine Wirtszelle nach Anspruch 13, **dadurch gekennzeichnet, daß** diese Wirtszelle Pichia pastoris ist.

16. Eine Wirtszelle nach Anspruch 13, **dadurch gekennzeichnet, daß** diese Wirtszelle pflanzlicher Natur ist.

17. Eine Wirtszelle nach Anspruch 16, **dadurch gekennzeichnet, daß** diese Wirtszelle eine Zellkultur von Tabak (Nicotiana) ist.

18. Ein pflanzlicher Wirtsorganismus, der einem eukaryotischen Expressionsvektor gemäß Anspruch 12 enthält, **dadurch gekennzeichnet, daß** dieser Wirtsorganismus eine Tabakpflanze (Nicotiana) ist.

19. Die Anwendung des rekombinanten Allergens Art v 1 nach Anspruch 9 für die Herstellung eines Mittels zur Diagnose und Therapie allergischer Patienten.

## Claims

1. Recombinant DNA molecules which code for the allergen Art v 1a which has the sequence shown in SEQ ID NO:2.

2. Recombinant DNA molecules as claimed in Claim 1, **characterised in that** these DNA molecules have the nucleotide sequence of SEQ ID NO: 1.

3. Recombinant DNA molecules as claimed in Claim 1, **characterised in that** these DNA molecules have a nucleotide sequence which has been derived from the amino acid sequence of SEQ ID NO:2 on the basis of the degeneration of the genetic code.

4. Recombinant DNA molecules as claimed in Claim 1 which have more than 60% sequence identity with SEQ ID NO:1.

5. Recombinant DNA molecules as claimed in Claim 4 which code for the isoforms Art v 1b and Art v 1c, **characterised in that** these isoforms have the amino acid sequences shown in SEQ ID NO:4 and 6.

6. Recombinant DNA molecules as claimed in Claim 5, **characterised in that** these DNA molecules have the nucleotide sequences shown in SEQ ID NO:3 and 5.

7. Recombinant DNA molecules as claimed in Claim 1 or 5, which hybridise under stringent conditions with the sequence shown in SEQ ID NO:1 and remain bonded by hybridisation under stringent washing conditions.

8. Recombinant DNA molecules as claimed in Claim 7, **characterised in that** the isoforms Art v 1b and Art v 1c which have the nucleotide sequences shown in SEQ ID NO:3 and 5 hybridise under stringent conditions with the An v 1A sequence shown in SEQ ID NO:1.

9. Method for the production of the Art v 1 allergen which comprises the following steps:
a) growing prokaryotic or eukaryotic host cells which contain a DNA coding for Art v 1 as claimed in Claim 2, 4 or 7, so that the Art v 1 allergen is expressed by the host cells;
b) isolation of the allergen Art v 1.

10. Method as claimed in Claim 9, **characterised in that** the recombinant allergen is glycosilated.

11. Method of producing a recombinant DNA as claimed in Claim 7, **characterised in that** 1 M NaCl in H₂O at 60°C is used as stringent hybridisation conditions and washing twice for 30 minutes at 50°C in 5x SSPE and 0.1% SDS, 1x SSPE = 0.18 M NaCl, 0.01M sodium phosphate pH 7.4, 1mM EDTA is used as stringent washing conditions.

12. Replicable prokaryotic or eukaryotic expression vector which contains DNA molecules as claimed in Claims 2, 4 or 7 as insert.

13. Prokaryotic or eukaryotic host cell which contains an expression vector as claimed in Claim 11.

14. Host cell as claimed in Claim 13, **characterised in that** this host cell is Escherichia coli.

15. Host cell as claimed in Claim 13, **characterised in that** this host cell is Pichia pastoris.

16. Host cell as claimed in Claim 13, **characterised in that** this host cell is vegetable in nature.

17. Host cell as claimed in Claim 16, **characterised in that** this host cell is a cell culture of tobacco (Nicotiana).

18. Vegetable host organism containing a eukaryotic expression vector as claimed in Claim 12, **characterised in that** this host organism is a tobacco plant (Nicotiana).

19. Use of the recombinant allergen Art v 1 as claimed in Claim 9 for the production of an agent for diagnosis and therapy of allergic patients.

## Revendications

1. Molécule recombinante d'ADN codant pour l'allergène Art v 1a qui possède la séquence décrite par SEQ ID N°:2.

2. Molécule recombinante d'ADN selon la revendication 1, **caractérisée en ce que** cette molécule d'ADN comprend la séquence de nucléotides selon SEQ ID N°:1.

3. Molécule recombinante d'ADN selon la revendication 1, **caractérisée en ce que** cette molécule comprend une séquence de nucléotides qui a été dérivée de la séquence d'acides aminés selon SEQ ID N°:2 en raison de la dégénérescence du code génétique.

4. Molécule recombinante d'ADN selon la revendication 1, qui comprend plus de 60 % d'identité de séquence avec la SEQ ID N°:1.

5. Molécule recombinante d'ADN selon la revendication 4, qui code pour les isoformes Art v 1b et Art v 1c, **caractérisée en ce que** ces isoformes comprennent les séquences d'acides aminés décrites selon SEQ ID N°:4 et 6.

6. Molécule recombinante d'ADN selon la revendication 5, **caractérisée en ce que** cette molécule d'ADN comprend les séquences de nucléotides décrites selon SEQ ID N°:3 et 5.

7. Molécule recombinante d'ADN selon la revendication 1 ou 5, qui, dans des conditions stringentes, s'hybride avec la séquence décrite selon SEQ ID N°:1, et demeure liée par hybridation dans des conditions stringentes de lavage.

8. Molécule recombinante d'ADN selon la revendication 7, **caractérisée en ce que** les isoformes Art v 1b et Art v 1c, qui comprennent les séquences de nucléotides décrites selon SEQ ID N°:3 et 5, s'hybrident, dans des conditions stringentes, avec la séquence Art v 1a décrite selon SEQ ID N°:1.

9. Méthode pour la production de l'allergène Art v 1, comprenant les étapes consistant à :
(a) cultiver des cellules hôtes procaryotiques ou eucaryotiques qui comprennent un ADN codant pour l'allergène Art v 1 selon la revendication 2, 4 ou 7, de telle sorte que l'allergène Art v 1 est exprimé par les cellules hôtes ;
(b) isoler l'allergène Art v 1.

10. Méthode selon la revendication 9, **caractérisée en ce que** l'allergène recombinant est glycolisé.

11. Procédé pour la fabrication d'un ADN recombinant selon la revendication 7, **caractérisé en ce que** 1 M NaCl dans H20 à 60°C est utilisé comme conditions stringentes d'hybridation et qu'un lavage à 50°C, 2 fois pendant 30 min, dans 5x SSPE et 0,1 % SDS. 1x SSPE = 0,18 M NaCl, 0,01 M phosphate de sodium pH = 7,4, 1 mM EDTA sont utilisés comme conditions stringentes de lavage.

12. Vecteur d'expression procaryotique ou eucaryotique apte à la réplication, qui comprend comme insert une molécule d'ADN selon la revendication 2, 4 ou 7.

13. Cellule hôte procaryotique ou eucaryotique, qui comprend un vecteur d'expression selon la revendication 11.

14. Cellule hôte selon la revendication 13, **caractérisée en ce que** cette cellule hôte est Escherichia coli.

15. Cellule hôte selon la revendication 13, **caractérisée en ce que** cette cellule hôte est Pichia pastoris.

16. Cellule hôte selon la revendication 13, **caractérisée en ce que** cette cellule hôte est de nature végétale.

17. Cellule hôte selon la revendication 16, **caractérisée en ce que** cette cellule hôte est une culture cellulaire du tabac (Nicotiana).

18. Organisme hôte végétal, qui comprend un vecteur d'expression eucaryotique selon la revendication 12, **caractérisé en ce que** cet organisme hôte est une plante de tabac (Nicotiana).

19. Utilisation de l'allergène recombinant Art v 1 selon la revendication 9, pour la fabrication d'un moyen permettant d'établir un diagnostic et une thérapie chez des patients allergiques.
